# EUROPEAN PATENT SPECIFICATION

(11) **EP 3 076 174 B1**
(45) Date of publication and mention of the grant of the patent: **10.07.2019**
(21) Application number: 16163074.4
(22) Date of filing: 31.03.2016
(51) Int. Cl.: G01N 33/49, G01N 27/327

(54) **ANALYTE METER WITH CONTOURED STRIP PORT TO IMPROVE ELECTROCHEMICAL TEST STRIP RELIABILITY**
ANALYTMESSGERÄT MIT PROFILIERTEM STREIFENPORT ZUR VERBESSERUNG DER ELEKTROCHEMISCHEN TESTSTREIFENZUVERLÄSSIGKEIT
APPAREIL DE MESURE D'UN ANALYTE AVEC ORIFICE DE BANDE PROFILÉE POUR AMÉLIORER LA FIABILITÉ DE LA BANDE D'ESSAI ÉLECTROCHIMIQUE

(30) Priority: 31.03.2015 US 201514674972
(43) Date of publication of application: 05.10.2016
(73) Proprietor: Roche Diabetes Care GmbH, 68305 Mannheim (DE); F. Hoffmann-La Roche AG, 4070 Basel (CH)
(72) Inventor: Riggles, Randall K, Indianapolis, IN 46236 (US); Sauers, Matthew C., Indianapolis, IN 46239 (US); Coburn, Caleb J., Carmel, IN 46033 (US); Uberta, III, Anthony J., Indianapolis, IN 46227 (US)
(74) Representative: Herzog, Fiesser & Partner Patentanwälte PartG mbB

(56) References cited:
- WO-A2-2007/086843
- US-A1- 2012 149 245

## Description

### FIELD

This disclosure relates to handheld *in vitro* analyte meters, such as a blood glucose meters, that use electrochemical test strips for analyte measurements.

### BACKGROUND

Diabetes mellitus, often referred to as diabetes, is a chronic condition in which a person has elevated blood glucose levels that result from the body's inability to produce insulin, use insulin, or both. There are three main types of diabetes. Type 1 diabetes usually strikes children and young adults and is linked to conditions such as autoimmune, genetic, environmental, or a combination. Type 2 diabetes accounts for 90-95% of diabetes cases and is linked to obesity and physical inactivity. Gestational diabetes is a form of glucose intolerance diagnosed during pregnancy and usually resolves soon after delivery.

In 2013, some 382 million people worldwide are estimated to have diabetes, and an estimated 5.1 million people between the ages of 20 and 79 die from diabetes annually, according to the International Diabetes Foundation Diabetes Atlas. In the United States, nearly 24 million Americans have diabetes with an estimated 25 percent of seniors age 60 and older being affected, according to The Centers for Disease Control and Prevention. Diabetes costs are estimated to be $174 billion in the United States alone every year, according to the National Diabetes Information Clearinghouse. Without treatment, diabetes can lead to severe complications such as heart disease, stroke, blindness, kidney failure, amputations, and death related to pneumonia and flu.

Blood glucose meters are used by persons with diabetes to measure blood glucose for the purpose of managing their blood glucose level according to therapeutic values typically through the use of insulin, medications, diet, exercise or a combination of these. The blood glucose meter user performs a glucose measurement by inserting a test strip into the blood glucose meter and placing a blood sample on a collection area of the test strip. Test strips typically include conductive materials deposited on a plastic substrate with some circuit traces exposed on the test strip end that is inserted into the blood glucose meter. When the blood glucose meter user inserts the test strip into the meter, the exposed circuit traces can become damaged resulting in a test strip that fails a self-test performed by the meter or possibly a blood glucose test performed that does not meet certain standards. An example of a blood glucose meter is described in Roche, Accu-Chek Nano Owner's Booklet for Self-Testing Only (2013). Other manufacturers of blood glucose meters include LifeScan, Inc. and Abbott Diabetes Care.

As an example, the document WO 2007/086843 A2 discloses a blood glucose measuring system including a lancet sensor assembly and a meter for use with the lancet sensor assembly. The lancet sensor assembly has a lancet member with a lance, lancet body having a drive wing extending outwardly from a side, and a sinuous portion, and an elongated carrier having a lancet member recess to contain the lancet member, a open end, a closed end, a side elongated opening for receiving the drive wing therethrough, and an anchoring member operatively connected to the end of the sinuous portion. The meter includes a measuring circuit, a lancet trigger, and a lancet driver where the lancet driver includes a driver piston engageable with the lancet trigger and a charging member that operatively engages with the driver piston to move the driver piston into an armed position and to stop the driver piston when released from the armed position.

As a further example, the document US 2012/149245 A1 discloses a modular analyte measurement system having a removable strip port module. In one embodiment, the analyte measurement system includes: an analyte meter; a removable strip port module; and a connector linking the removable strip port module to the analyte meter. The analyte meter includes: a meter housing; a receptacle formed in the meter housing; a processing circuit disposed within the housing; and an input interface within the receptacle and electrically coupled to the processing circuit. The removable strip port module includes: a module housing sized to at least partially fit within the receptacle of the analyte meter; an analyte test strip port disposed within the module housing to receive an analyte test strip via an aperture formed in the module housing; and an output interface coupled to the analyte test strip port. The connector links the output interface with the input interface.

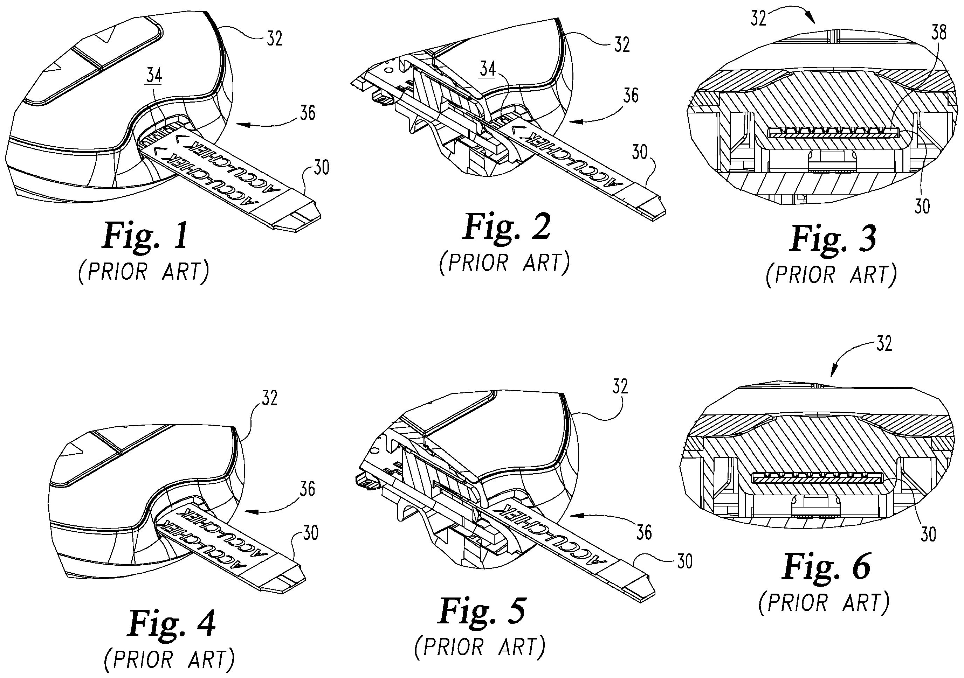

Prior art FIGS. 1-6 show a test strip 30 being inserted into a blood glucose meter 32. A person with diabetes can have difficulty inserting the test strip 30 into the blood glucose meter 32 due to disabilities such as diminished eyesight, neuropathy, and movement disorders. Prior art FIGS. 1-3 show the test strip 30 at the initial insertion point with exposed circuit traces 34 entering the strip port 36 that has a clearance 38 shown in FIG. 3 between the test strip top with exposed circuit traces 34 and the strip port top. The exposed circuit traces 34 are susceptible damage such as scratching across a circuit trace 34 causing an open circuit. The exposed circuit traces 34 are thin and easily scratched upon frictional contact and deposited on plastic and can easily delaminated from the plastic. Movement of the test strip 30 when in the initial insertion position can cause the exposed circuit traces 34 to frictionally engage the strip slot top potentially resulting in damage to the exposed circuit traces 34. Prior art FIGS. 4-6 show the test strip 30 fully inserted with the test strip 30 filling the strip port due because the test strip top covering has entered the strip slot. The exposed circuit traces 34 are no longer frictionally engaging the strip port top.

What is needed is an analyte meter, such as a blood glucose meter, that is configured to prevent or reduce damage to exposed test strip circuit traces during insertion of test strips into the meter strip port.

### SUMMARY

In one embodiment an analyte meter, such as a blood glucose meter, a coagulation meter, and a cardiac disease risk factors meter, with contoured strip port to improve electrochemical test strip reliability comprises an analyte meter that comprises, a housing carrying a circuit board, a meter processor coupled to the circuit board, memory coupled to the circuit board and connected to the meter processor, a display connected to the meter processor, a measurement processor connected to meter processor, a strip connector connected to the measurement processor, the strip connector having connector terminals, a contoured strip port carried in the housing and located next to the strip connector, the contoured strip port can be attached to a circuit board and comprises, a strip shelf having side guides for aligning a test strip with the contoured strip port, a strip port opening that comprises an opening bottom extending from a first bottom edge to a second bottom edge, top guides located at a first top edge and a second top edge, and a contoured top extending between the top guides creating an arched clearance above the opening bottom to provide clearance for test strip electrical traces. The contoured strip port has surface roughness of A2 from Society of the Plastics Industry surface roughness of about 1 µm to about 2 µm.

The above embodiment can have top guides with a strip engagement surface that is substantially flat and parallel to the opening bottom. The strip engagement surface can have a width sufficient to engage a strip first edge to prevent the strip first edge from engaging a contoured top.

The above embodiment can further comprise a contoured face above the contoured top to reduce friction upon contact with the test strip. The contoured face is convex and extends over a portion of the strip shelf.

The above embodiment can further comprise a test strip having a strip contact end and a strip dosing end, the test strip comprises a first substrate having a first substrate top and a first substrate bottom, a strip contact area on the first substrate top near the strip contact end, the strip contact area having a plurality of strip contacts, electrical traces formed on the first substrate top connecting the strip contacts to electrodes located near the strip dosing end, a second substrate attached over the first substrate having a second substrate top extending from the strip dosing end to a second substrate contact end terminating a predetermined distance from the strip contacts end, a dosing site formed on the first substrate bottom at the strip dosing end, the dosing site covered by the second substrate top and coupled to the electrodes, a strip first edge formed on first substrate top extending from the strip contact end to the second substrate contacts end, and, a strip second edge formed on the second substrate top extending from the second substrate contacts end to the strip dosing end. The strip first edge can be free from electrical traces. The electrical traces have electrical segments that are curvilinear. The electrical traces have a trace width in the range from about 100 µm to about 250 µm. The electrical traces are sputtered on the first substrate top and selected from gold, palladium, and gold and palladium.

In another embodiment, the contoured strip port for an analyte meter to improve electrochemical test strip reliability comprises a contoured strip port carried in the housing and located next to the strip connector. The contoured strip port comprises a strip shelf having side guides for aligning the test strip with the contoured strip port, a strip port opening that comprises an opening bottom extending from a first bottom edge to a second bottom edge, top guides located at a first top edge and a second top edge, a contoured top extending between the top guides creating an arched clearance above the opening bottom to provide clearance for test strip electrical traces. The top guides have a strip engagement surface that is substantially flat and parallel to the opening bottom. The strip engagement surface has a width sufficient to engage a strip first edge to prevent the strip first edge from engaging the contoured top. The contoured strip can further comprising a contoured slot face above the contoured top to reduce friction upon contact with the test strip. The contoured slot face is convex and extends over the strip shelf. The contoured strip port has surface roughness of A2 from Society of the Plastics Industry surface roughness of about 1 µm to about 2 µm.

Furthermore, a method is described for inserting a test strip into a handheld analyte meter to improve the reliability of an electrochemical test strip. The method comprises placing a test strip contacts end on a strip shelf having side guides, moving the test strip contacts end toward a strip port opening, guiding the test strip contacts end with the strip shelf and side guides toward the strip port opening, inserting the test strip contacts end into the strip port opening, engaging a strip first edge with top guides to maintain alignment of the strip contact end with a contoured top, avoiding contact between electrical traces formed on the first substrate top and the contoured top, engaging a strip second edge with top guides, after further insertion, to maintain alignment of the strip contact end with a strip connector; inserting the a strip contact area into the strip connector; and, connecting strip contacts with terminal contacts as the test strip contacts end seats in the strip connector. This embodiment can further comprise bending the test strip axially during insertion into the contoured strip port causing the electrical tracts to engage the contoured face.

To summarize, the present invention relates to a strip port according to independent claim 1. The dependent claims relate to advantageous details.

### BRIEF DESCRIPTION OF THE DRAWINGS

FIG. 1 (prior art) shows a test strip port isometric view with a test strip at an initial insertion position;
FIG. 2 (prior art) shows a test strip slot axial cross section isometric view with a test strip at an initial insertion position that corresponds to FIG. 1;
FIG. 3 (prior art) shows a test strip slot transverse cross section view with a test strip at an initial insertion position that corresponds to FIGS. 1-2;
FIG. 4 (prior art) shows a test strip port isometric view with a test strip in an inserted position;
FIG. 5 (prior art) shows a test strip slot axial cross section isometric view with a test strip in an inserted position that corresponds to FIG. 4;
FIG. 6 (prior art) shows a test strip slot transverse cross section view with a test strip at an inserted position that corresponds to FIGS. 4-5;
FIG. 7 shows a person with diabetes in a self-testing environment;
FIG. 8 shows an analyte meter;
FIG. 9 shows a test strip;
FIG. 10 shows an analyte meter electrical schematic;
FIG. 11 shows a contoured strip port component;
FIG. 12 shows another embodiment of a contoured strip port component;
FIG. 13 shows the contoured strip port component of FIG. 11 installed on a circuit board;
FIG. 14 shows the opposite side of the contoured strip port and circuit board of FIG. 13;
FIG. 15 shows a front view of a contoured strip port;
FIG. 16 shows a top view of a contoured strip port;
FIG. 17 shows an isometric view of a contoured strip port;
FIG. 18 shows a contoured strip port with a test strip positioned on a contoured strip port platform;
FIG. 19 shows a contoured strip port with a test strip partially inserted into strip port opening;
FIG. 20 shows a front sectioned view of a contoured strip port with a test strip positioned as in FIG. 19;
FIG. 21 shows a sectioned view of a contoured strip port with a test strip fully inserted into the strip port opening;
FIG. 22 shows a front sectioned view of a contoured strip port with a test strip positioned as in FIG. 21;
FIG. 23 shows a sectioned view of a contoured opening with a test strip partially inserted;
FIG. 24 shows a contoured strip port with a test strip bent from improper insertion; and
FIG. 25 shows a flow chart of a method for inserting a test strip into a handheld analyte meter.

### DETAILED DESCRIPTION

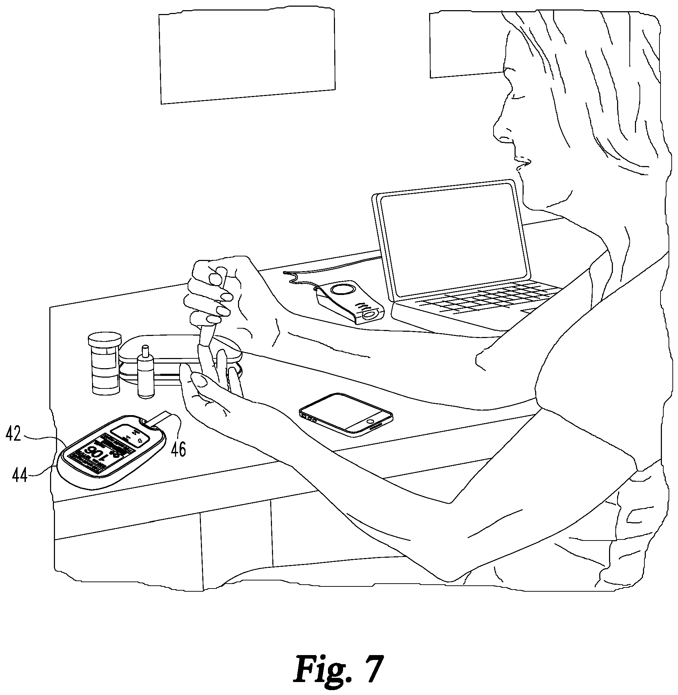

FIG. 7 shows a person with diabetes 40 in a self-testing environment such as in a home. The self-testing environment includes an analyte meter 42, such as a blood glucose meter 44, test strip container, test strip, and lancet. The person with diabetes 40 is typically the user of the blood glucose meter 42; however, the user can also be a clinician, healthcare provider, family member or other person. The meter 44 is operated by inserting a disposable test strip 46 into the meter 44. Test strips 46 can be fragile particularly the exposed electrical traces that can be damaged during test strip 46 insertion causing the strip to fail a self-test or cause create other errors. The user lances typically a finger to obtain a small blood sample that is placed on the test strip dosing area. The meter 42 performs electrochemical analysis of the blood sample and displays a blood glucose measurement. The blood glucose measurement is used for therapy decisions such as insulin dosage and carbohydrate consumption. The person with diabetes 40 can have disabilities such as diminished eyesight, neuropathy, and movement disorders that can make inserting a test strip into the analyte meter difficult. In addition to blood glucose meters 44, other analyte meters 42 used in self-testing and point-of-care environments include coagulation meters that measure prothrombin time PT and international normalized ratio INR, and cardiovascular disease factor meters that measure total cholesterol, triglycerides, and blood glucose.

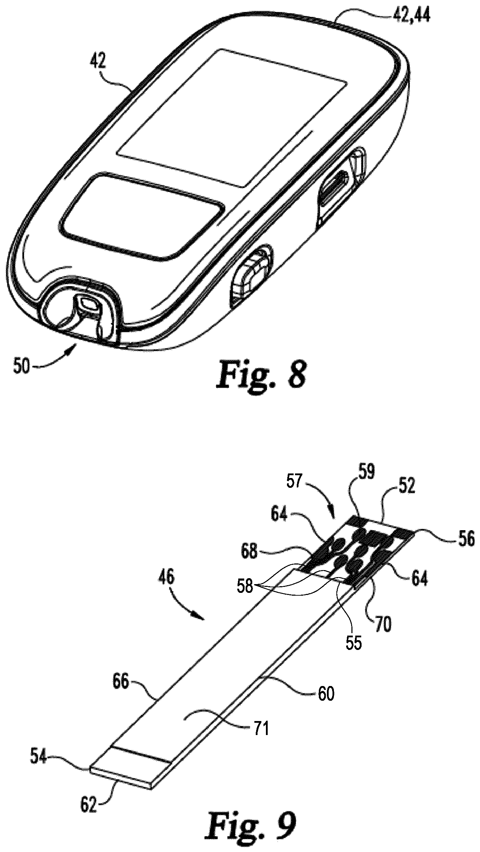

FIG. 8 shows an analyte meter 42 in the form of a blood glucose meter 44 with a contoured strip port 50 to improve electrochemical test strip reliability. The blood glucose meter 44 comprises a housing 48 having a contoured strip port 50. A glucose meter 44 with a test strip ejection mechanism is shown in U.S. Publication No. 2014/0005507 A1, Test Strip Ejector For Medical Device (January 2, 2014) assigned to Roche Diagnostics Operations, Inc., which is hereby incorporated by reference.

FIG. 9 shows a test strip 46. The test strip 46 has a strip contact end 52 and a strip dosing end 54 and comprises a first substrate 56, a strip contact area 57, electrical traces 58, a second substrate 60, a dosing site 62, a strip first edge 64, and a strip second edge 66. The electrochemical test strips 46 can be fragile particularly in regard to exposed electrical traces 58 that are often made from soft metals in widths and depths measured in microns and sputtered on flexible plastic materials with limited adhesion for the deposited metals. The electrical traces 58 are composed of a material with having limited interference with enzymatic reactions such as gold, palladium, or gold and palladium that are sputtered on plastic. The distal electrical traces 58 have electrical segments that are curvilinear, a trace width in the range from about 100 µm to about 250 µm. The electrical traces 58 that are substantially transverse to the test strip are particularly vulnerable to damage because even a small scratch axial scratch and create a faulty or open circuit.

The first substrate 56 has a first substrate top 68 and a first substrate bottom 70 and is formed from a flexible plastic such as polyethylene. A strip contact area 57 is located on the first substrate top 68 at the strip contact end 52 to provide a plurality of strip contacts 59 such as eight strip contacts 59. Electrical traces 58 are formed on the first substrate top 68 connecting strip contacts to electrodes located on the strip dosing end 54. The strip contact end 52 further comprises non-evident coding traces (not shown). Selected non-evident coding traces are cut during manufacturing to change their impedance to create a non-evident calibration code for the characteristics of the dosing site enzyme. The strip contact area 57 is not covered by the second substrate 60 that exposes the strip contacts 59, certain electrical traces 58, and certain non-evident coding traces to potential damage particularly during test strip 46 insertion.

The second substrate 60 is attached over the first substrate 56 and can be manufactured from a material similar to the first substrate 56. The second substrate 60 has a second substrate top 71 extending from the strip dosing end 54 to a second substrate contact end terminating a predetermined distance from the strip contacts 59. A dosing site 62 is formed on the first substrate bottom 70 at the dosing site 62 with an enzyme and electrodes that are connected by electrical traces 58 to the strip contacts 59. The dosing site 62 can be covered or partially covered by the second substrate 60.

The strip first edges 64 are formed at the intersection of the first substrate top 68 and first substrate side edges and extend from the first substrate contact end 52 to the second substrate 60 contact end that terminates a predetermined distance from the strip contacts 59. The strip second edges 66 are formed at the intersection of the second substrate top 71 and the second substrate edges and extend from the second substrate contact end 55 to the second substrate dosing end 54. The strip first edges 64 are free from electrical traces 58, although there is some material that looks like an electrical trace 58 but is actually material that is incidental to the manufacturing process. The strip first edges 64 are free from electrical traces 58 because the strip first edges 64 can have frictional contact with the contoured strip port 50.

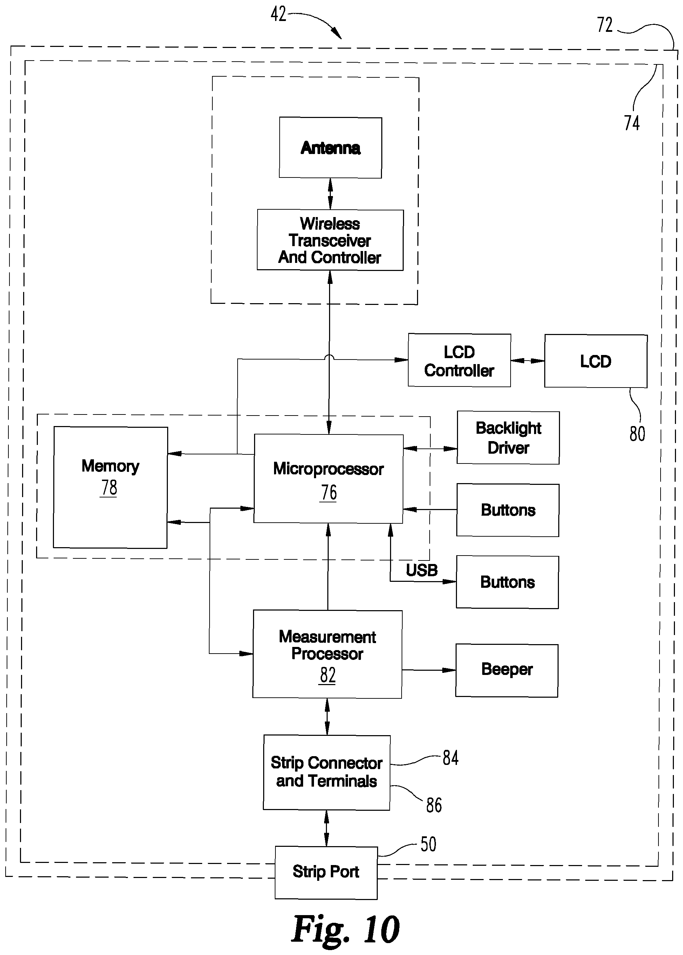

FIG. 10 shows an analyte meter 42 electrical schematic. The analyte meter 42 comprises a housing 72 carrying a circuit board 74, a meter processor 76 coupled to the circuit board 74, memory 78 coupled to the circuit board 74 and connected to the processor 76, a display 80 connected to the meter processor 76, a measurement processor 82 (also known as a measurement engine) connected to the meter processor 76, a strip connector 84 connected to the measurement processor 82 with the strip connector 84 having connector terminals 86, and a contoured strip port 50.

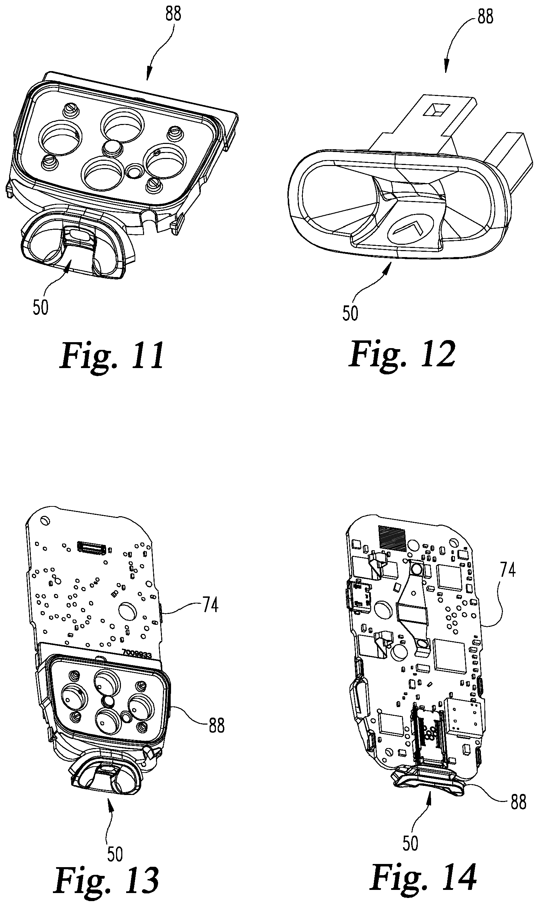

FIG. 11 shows contoured strip port component 88, and FIG. 12 shows another embodiment of the contoured strip port component 88. The contoured strip port component 88 is manufactured from a plastic such as a polycarbonate and more specifically polycarbonate acrylonitrile butadiene styrene (PC-ABS) that is finished to a surface roughness of A2 from Society of the Plastics Industry that is equivalent to a surface roughness of about 1 µm to about 2 µm.

FIGS. 13 and 14 shows the contoured strip port component 88 installed on a circuit board 74. In FIG. 13, the contoured strip port component 88 has details that engage details on the printed circuit board 74 top side to locate and secure the contoured strip port component 88 to the circuit board 74. In FIG. 14, contoured strip port component 88 installed on the circuit board 74 showing the circuit board 74 bottom side. The contoured strip port component 88 is carried in the housing 72 and is located next to and aligned with the strip connector 84.

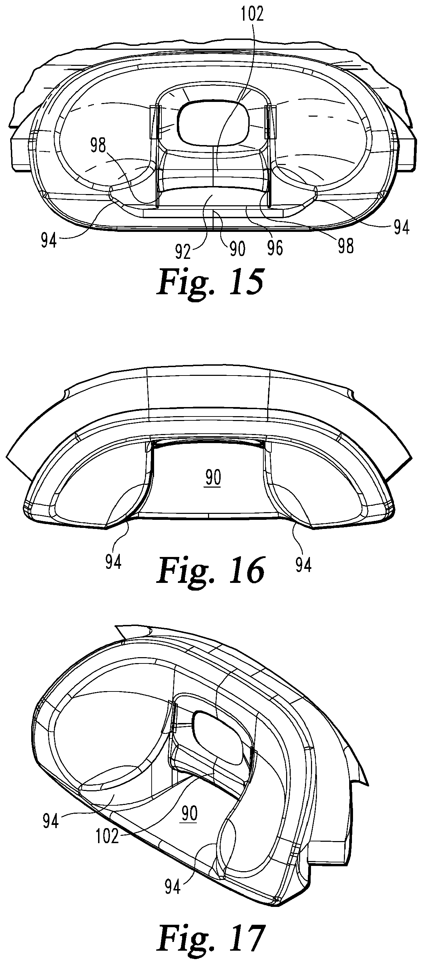

FIG. 15 shows a front view of the contoured strip port 50 , FIG. 16 shows a top view of the contoured strip port 50, and FIG. 17 shows an isometric view of the contoured strip port 50. The contoured strip port 50 comprises a strip shelf 90 and a strip port opening 92. The strip shelf 90 has side guides 94 for aligning the test strip 46 with the strip port opening 92 prior to the test strip 46 entering the strip port opening 92. The strip shelf 90 functions to correct for certain test strip 46 insertion angle variances by guiding the test strip 46 into the strip port opening 92.

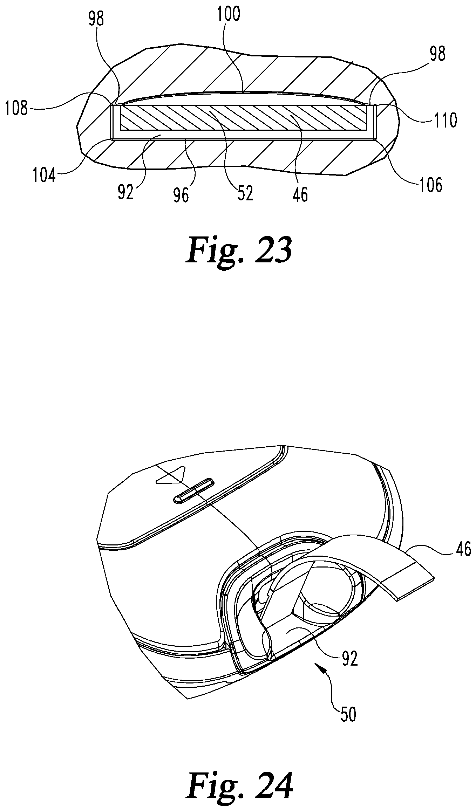

The strip port opening 92 comprises an opening bottom 96 , top guides 98, a contoured top 100, and a contoured face 102. The opening bottom 96 extends from a first bottom edge 104 to a second bottom edge 106. The top guides 98 (also FIG. 23 shows the top guides enlarged) are located at a first top edge 108 and a second top edge 110. The top guides 98 have a strip engagement surface that is substantially flat and parallel to the opening bottom 96. The strip engagement surface is sufficient to engage a strip first edge 64 to prevent the strip first edge 64 from engaging the contoured top 100 such as a width of about 0.1 mm. The contoured top 100 extends between the top guides 98 creating an arched clearance above the opening bottom 96 to provide clearance for test strip electrical traces 58. The contoured slot face 102 above the contoured top 100 reduces friction upon contact with the test strip 46.

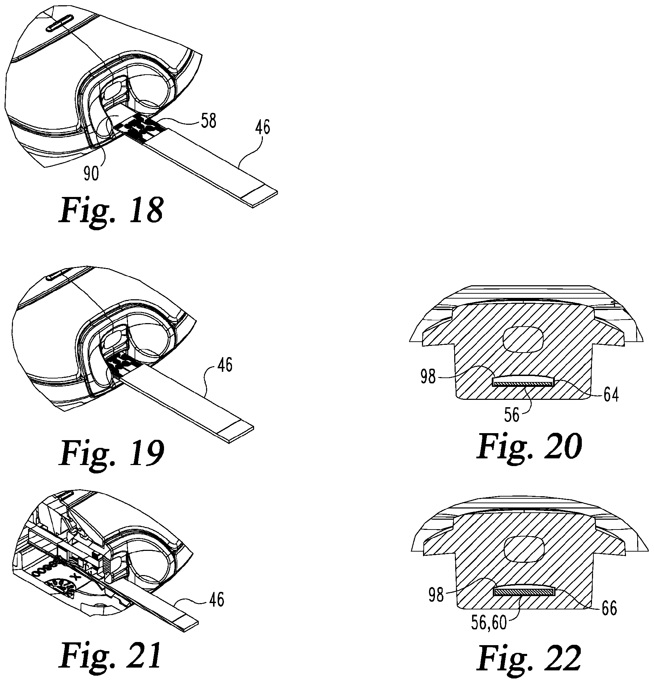

FIG. 18 shows a test strip 46 on the strip shelf 90 being guided toward the strip port opening 92.

FIGS. 19 shows a test strip 46 entering the strip port opening 92 with the first substrate 56 positioned in the strip port opening, and FIG. 20 shows a cross-section view of FIG. 19. The strip first edge 64 has been guided by the strip shelf 90, so that strip first edge 64 is not engaging the top guides 98.

FIGS. 21 shows a test strip 46 inserted into the strip port opening 92 with the first substrate 56 and second substrate 60 positioned in the strip port opening 92, and FIG. 22 shows a cross-section view of FIG. 21. The strip second edge 66 is engaging the top guides 98.

FIG. 23 shows a sectioned view of a contoured strip port 50 with a test strip 46 partially inserted so that the first substrate 56 is within the strip port opening 92, but the second substrate 60 is not within the strip port opening 92. With only the first substrate 56 inserted into the strip port opening 92 there is an open space that will be occupied by the second substrate 60 when the test strip 46 is fully inserted. The open space has permitted the test strip 46 to move up towards the contoured top 100, but the strip first edge 64 has contacted the top guides 98 to prevent the test strip 46, and particularly the electrical traces 58, from potentially contacting the contoured top 100. The contoured top 100 also provides space to reduce the opportunity for the electrical traces 58 to contact the contoured top 100.

FIG. 24 shows a contoured strip port 50 with a test strip 46 bent from improper insertion. The test strip first substrate 56 and second substrate 60 are plastic, to the test strip 46 is subject to bending, particularly axially. If a user does not guide the test strip 46 in a coplanar path along the strip shelf 92, but engages the strip contacts end 52 on the strip shelf 92 with sufficient force, then the strip contact end 52 can catch on the strip shelf 92 causing the test strip 46 to axially bend (also known as porpoising). The electrical traces 58 can contact the contoured face 102 (FIG. 17). The contoured face 102 is radiused to reduce frictional contact with the electrical traces 58 which reduces the risk that the electrical traces 58 will become damaged.

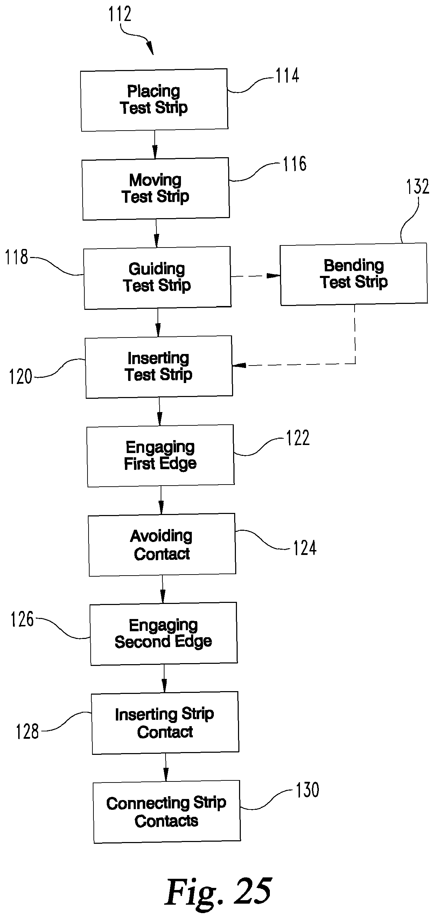

FIG. 25 shows a flow chart of a method 112 for inserting a test strip 46 into a handheld analyte meter 42 to improve the reliability of an electrochemical test strip 46. The method 112 comprises the following steps. Placing 114 a test strip contacts end 52 on a strip shelf 90 having side guides 94. Moving 116 the test strip contacts end 52 toward a strip port opening 92. Guiding the test strip contacts end 52 with the strip shelf 90 and side guides 92 toward the strip port opening 92. Inserting 120 the test strip contacts end 52 into the strip port opening 92. Engaging 122 a strip first edge 64 with top guides 98 to maintain alignment of the strip contact end 52 with a contoured top 100. Avoiding 124 frictional contact between electrical traces 58 formed on the first substrate top 68 and the contoured top 100. Engaging 126 a strip second edge 66 with top guides 98, after further insertion, to maintain alignment of the strip contact end 52 with a strip connector 84. Inserting 128 the strip contact area 57 into the strip connector 84. Connecting 130 strip contacts 59 with terminal 86 contacts as the test strip contacts end 52 seats in the strip connector 84. Some embodiments can also include the step of bending 132 the test strip 46 axially during insertion into the contoured strip port 50 causing the electrical traces 58 to engage the contoured face 102. The contoured face 102 reduces frictional contact between the electrical traces 58 and the contoured face 102 to reduce the risk that the electrical traces 58 will become damaged.

Table 1 shows test data comparing performance of a slotted strip port (prior art FIGS. 1-6) with a contoured strip port 50. The testing was performed on 64 blood glucose meters 44 such as shown in FIG. 8 configured with a rectangular strip port and 64 blood glucose meter 44 such as shown in FIG. 8 configured with a contoured strip port 50 installed in test stands with each test stand holding up to 16 meters 44. The test strips 46 used were standard pilot production test strips 46 that met normal manufacturing standards. The test strips 46 were inserted into the meters 44 by a laboratory technician using normal insertion technique that resulted in some insertion angle variation and an occasional bending or porpoising, such as shown in FIG. 24, during insertion. The testing produced two types of errors. The first type of error recorded was a "no strip recognition error" that results when a meter does not recognize that a strip has been inserted because the initial strip continuity test failed. The "no strip recognition error" was recorded by the laboratory technician because the meter is not capable or recording this type of error. The second type of error resulted in an error code from the failure of various test strip diagnostics. The test data demonstrates that the contoured strip port 50 significantly improved the reliability of the test strips 46 over the prior art slotted strip port.

**TABLE 1**

| *Strip Port* | *Total Strips Tested* | *Number of Errors* | *Error Rate* |
|---|---|---|---|
| *Slotted (prior art)* | 4,262 | 136 | 3.19% |
| *Contoured* | 7,395 | 10 | 0.135% |

Thus, embodiments of the analyte meter with contoured strip port to improve electrochemical test strip reliability are disclosed. One skilled in the art will appreciate that the teachings can be practiced with embodiments other than those disclosed. The disclosed embodiments are presented for purposes of illustration and not limitation, and the invention is only limited by the claims that follow.

## Claims

1. A contoured strip port (50) for an analyte meter (42) to improve test strip reliability, wherein the contoured strip port (50) is adapted to be carried in a housing (72) of the analyte meter (42) and to be located next to a strip connector (84) of the analyte meter (42), wherein the contoured strip port (50) comprises:
a strip shelf (90) having side guides (94) for aligning a test strip (46) with the strip port (50),
a strip port opening (92) that comprises
an opening bottom (96) extending from a first bottom edge (104) to a second bottom edge (106), **characterized in that** it further comprises top guides (98) located at a first top edge (108) and a second top edge (110), and
a contoured top (100) extending between the top guides (98) creating an arched clearance above the opening bottom (96) to provide clearance for test strip electrical traces (58),
wherein the top guides (98) have a strip engagement surface that is substantially flat and parallel to the opening bottom (96), wherein the strip engagement surface has a width sufficient to engage a strip first edge (64) of the test strip (46) to prevent the strip first edge (64) from engaging the contoured top (100).

2. The contoured strip port (50) according to the preceding claims, wherein the strip engagement surface has a width of about 0.1 mm.

3. The contoured strip port (50) according to any one of the preceding claims, further comprising a contoured slot face (102) above the contoured top (100) to reduce friction upon contact with the test strip (46).

4. The contoured strip port (50) according to the preceding claim, wherein the contoured slot face (102) is convex and extends over the strip shelf (90).

5. The contoured strip port (50) according to any one of the preceding claims, wherein the contoured strip port (50) has surface roughness of A2 from Society of the Plastics Industry surface roughness of about 1 µm to about 2 µm.

6. The contoured strip port (50) according to any one of the preceding claims, wherein the strip port opening (92) is designed such that a test strip (46) is insertable into the strip port opening (92), wherein the test strip (46) has a strip contact end (52) and a strip dosing end (54), the test strip (46) comprises,
a first substrate (56) having a first substrate top (68) and a first substrate bottom (70),
a strip contact area (57) on the first substrate top (68) near the strip contact end (52), the strip contact area (57) having a plurality of strip contacts (59),
electrical traces (58) formed on the first substrate top (68) connecting the strip contacts (59) to electrodes located near the strip dosing end (54),
a second substrate (60) attached over the first substrate (56) having a second substrate top (71) extending from the strip dosing end (54) to a second substrate contact end terminating a predetermined distance from the strip contacts (59) end,
a dosing site (62) formed on the first substrate bottom (70) at the strip dosing end (54), the dosing site (62) covered by the second substrate top (71) and coupled to the electrodes,
a strip first edge (64) formed on first substrate top (68) extending from the strip contact end (52) to the second substrate contacts end (55), and
a strip second edge (66) formed on the second substrate top (71) extending from the second substrate contacts end (55) to the strip dosing end (54).

## Patentansprüche

1. Profilierter Streifenport (50) für ein Analytmessgerät (42) zur Verbesserung der Teststreifenzuverlässigkeit, wobei der profilierte Streifenport (50) eingerichtet ist, um in ein Gehäuse (72) des Analytmessgeräts (42) aufgenommen zu werden und neben einem Streifenanschluss (84) des Analytmessgeräts (42) positioniert zu sein, wobei der profilierte Streifenport (50) umfasst:
einen Streifensockel (90), aufweisend seitliche Führungen (94) zum Ausrichten eines Teststreifens (46) mit dem Streifenport (50),
eine Streifenport-Öffnung (92), die umfasst:
einen Öffnungsboden (96), verlaufend von einer ersten unteren Kante (104) zu einer zweiten unteren Kante (106), **dadurch gekennzeichnet, dass** er ferner umfasst:
obere Führungen (98), positioniert an einer ersten oberen Kante (108) und einer zweiten oberen Kante (110), und
ein profiliertes Oberteil (100), verlaufend zwischen den oberen Führungen (98), erzeugend eine Bogenöffnung oberhalb des Öffnungsbodens (96) zum Bereitstellen einer Öffnung für elektrische Teststreifenleiter (58),
wobei die oberen Führungen (98) eine Streifeneingriffsfläche aufweisen, die im Wesentlichen flach und parallel zu dem Öffnungsboden (96) ist, wobei die Streifeneingriffsfläche eine Breite aufweist, die ausreichend ist, um eine erste Streifenkante (64) eines Teststreifens (46) zu erfassen, um den Eingriff der ersten Teststreifenkante (64) mit dem profilierten Oberteil (100) zu verhindern.

2. Profilierter Streifenport (50) nach dem vorgehenden Anspruch, wobei die Streifeneingriffsfläche eine Breite von etwa 0,1 mm aufweist.

3. Profilierter Streifenport (50) nach einem der vorgehenden Ansprüche, ferner umfassend eine profilierte Schlitzseite (102) oberhalb des profilierten Oberteils (100) zum Reduzieren von Reibung bei Kontakt mit dem Teststreifen (46).

4. Profilierter Streifenport (50) nach dem vorgehenden Anspruch, wobei die profilierte Schlitzseite (102) konvex ist und über den Streifensockel (90) verläuft.

5. Profilierter Streifenport (50) nach einem der vorgehenden Ansprüche, wobei der profilierte Streifenport (50) eine Oberflächenrauheit A2 nach der Oberflächenrauheit der "Society of the Plastics Industry" von etwa 1 µm bis etwa 2 µm aufweist.

6. Profilierter Streifenport (50) nach einem der vorgehenden Ansprüche, wobei die Streifenport-Öffnung (92) so konzipiert ist, dass ein Teststreifen (46) in die Streifenport-Öffnung (92) einsetzbar ist, wobei der Teststreifen (46) ein Streifenkontaktende (52) und ein Streifen-Dosierende (54) aufweist und der Teststreifen (46) umfasst:
ein erstes Substrat (56), aufweisend ein erstes Substrat-Oberteil (68) und ein erstes Substrat-Unterteil (70),
eine Streifenkontaktfläche (57) auf dem ersten Substrat-Oberteil (68) nahe dem Streifenkontaktende (52), wobei die Streifenkontaktfläche (57) eine Mehrzahl von Streifenkontakten (59) aufweist,
elektrische Leiter (58), ausgebildet auf dem ersten Substrat-Oberteil (68), verbindend die Streifenkontakte (59) mit nahe dem Streifen-Dosierende (54) positionierten Elektroden,
ein zweites Substrat (60), angebracht über dem ersten Substrat (56), aufweisend ein zweites Substrat-Oberteil (71), verlaufend von dem Streifen-Dosierende (54) zu einem zweiten Substrat-Kontaktende, das eine vorbestimmte Distanz von dem Ende der Streifenkontakte (59) abschließt,
eine Dosierstelle (62), ausgebildet auf dem ersten Substat-Unterteil (70) am Streifen-Dosierende (54), wobei die Dosierstelle (62) durch das zweite Substrat-Oberteil (71) bedeckt und mit den Elektroden gekoppelt ist,
eine erste Streifenkante (64), ausgebildet auf dem ersten Substrat-Oberteil (68), verlaufend von dem Streifenkontaktende (52) zu dem zweiten Substrat-Kontaktende (55) und
eine zweite Streifenkante (66), ausgebildet auf dem zweiten Substrat-Oberteil (71), verlaufend von dem zweiten Substrat-Kontaktende (55) zu dem Streifen-Dosierende (54).

## Revendications

1. Port de bandelette profilé (50) pour un appareil de mesure d'analytes (42) pour améliorer la fiabilité d'une bandelette réactive, le port de bandelette profilé (50) étant adapté pour être porté dans un boîtier (72) de l'appareil de mesure d'analytes (42) et pour être situé à côté d'un connecteur de bandelette (84) de l'appareil de mesure d'analytes (42), le port de bandelette profilé (50) comprenant :
une plage d'accueil de bandelette (90) ayant des guides latéraux (94) pour aligner une bandelette réactive (46) avec le port de bandelette (50),
une ouverture de port de bandelette (92) qui comprend un dessous d'ouverture (96) s'étendant d'un premier bord inférieur (104) à un deuxième bord inférieur (106), **caractérisée en ce qu'**elle comprend en outre
des guides supérieurs (98) situés à un premier bord supérieur (108) et un deuxième bord supérieur (110), et
un dessus profilé (100) s'étendant entre les guides supérieurs (98) créant un espace libre arqué au-dessus du dessous d'ouverture (96) pour fournir un espace pour des traces électriques de bandelette réactive (58),
dans lequel les guides supérieurs (98) ont une surface de prise de bandelette qui est sensiblement plate et parallèle au dessous d'ouverture (96), dans lequel la surface de prise de bandelette a une largeur suffisante pour entrer en prise avec un premier bord de bandelette (64) de la bandelette réactive (46) pour empêcher le premier bord de bandelette (64) d'entrer en prise avec le dessus profilé (100).

2. Port de bandelette profilé (50) selon la revendication précédente, dans lequel la surface de prise de bandelette a une largeur d'environ 0,1 mm.

3. Port de bandelette profilé (50) selon l'une quelconque des revendications précédentes, comprenant en outre une face fendue profilée (102) sur le dessus profilé (100) pour réduire le frottement lors d'un contact avec la bandelette réactive (46).

4. Port de bandelette profilé (50) selon la revendication précédente, dans lequel la face fendue profilée (102) est convexe et s'étend par-dessus la plage d'accueil de bandelette (90).

5. Port de bandelette profilé (50) selon l'une quelconque des revendications précédentes, le port de bandelette profilé (50) ayant une rugosité de surface A2 d'après une rugosité de surface de la Society of the Plastics Industry d'environ 1 µm à environ 2 µm.

6. Port de bandelette profilé (50) selon l'une quelconque des revendications précédentes, l'ouverture de port de bandelette (92) étant conçue de telle sorte qu'une bandelette réactive (46) peut être insérée dans l'ouverture de port de bandelette (92), la bandelette réactive (46) ayant une extrémité de contact de bandelette (52) et une extrémité de dosage de bandelette (54), la bandelette réactive (46) comprenant
un premier substrat (56) ayant un premier dessus de substrat (68) et un premier dessous de substrat (70),
une zone de contact de bandelette (57) sur le premier dessus de substrat (68) à côté de l'extrémité de contact de bandelette (52), la zone de contact de bandelette (57) ayant une pluralité de contacts de bandelette (59),
des traces électriques (58) formées sur le premier dessus de substrat (68) reliant les contacts de bandelette (59) à des électrodes situées près de l'extrémité de dosage de bandelette (54),
un deuxième substrat (60) attaché sur le premier substrat (56) ayant un deuxième dessus de substrat (71) s'étendant de l'extrémité de dosage de bandelette (54) à une deuxième extrémité de contact de substrat se terminant à une distance prédéterminée de l'extrémité des contacts de bandelette (59),
un site de dosage (62) formé sur le premier dessous de substrat (70) à l'extrémité de dosage de bandelette (54), le site de dosage (62) étant recouvert par le deuxième dessus de substrat (71) et couplé aux électrodes,
un premier bord de bandelette (64) formé sur le premier dessus de substrat (68) s'étendant de l'extrémité de contact de bandelette (52) à la deuxième extrémité de contact de substrat (55), et
un deuxième bord de bandelette (66) formé sur le deuxième dessus de substrat (71) s'étendant de la deuxième extrémité de contact de substrat (55) à l'extrémité de dosage de bandelette (54).
